# EUROPÄISCHE PATENTSCHRIFT

(11) **EP 2 285 488 B1**
(45) Veröffentlichungstag und Bekanntmachung des Hinweises auf die Patenterteilung: **08.02.2012**
(21) Anmeldenummer: 09761559.5
(22) Anmeldetag: 07.05.2009
(51) Int. Cl.: B01J 23/44, B01J 21/04, B01J 35/10, C07C 29/00

(54) **VERFAHREN ZUR HERSTELLUNG VON GESÄTTIGTEN ETHERN DURCH HYDRIERUNG UNGESÄTTIGTER ETHER**
METHOD FOR PRODUCING SATURATED ETHERS BY HYDROGENATION OF UNSATURATED ETHERS
PROCÉDÉ DE PRÉPARATION D'ÉTHERS SATURÉS PAR HYDRATATION D'ÉTHERS INSATURÉS

(30) Priorität: 11.06.2008 DE 102008002347
(43) Veröffentlichungstag der Anmeldung: 23.02.2011
(73) Patentinhaber: Evonik Oxeno GmbH, 45772 Marl (DE)
(72) Erfinder: STOCHNIOL, Guido, 45721 Haltern am See (DE); SANTIAGO FERNANDEZ, Silvia, E-33009 Oviedo (ES); NIERLICH, Franz, 45768 Marl (DE); HOUBRECHTS, Stephan, B-2570 Duffel (BE); BÜSCHKEN, Wilfried, 45721 Haltern am See (DE)
(86) Internationale Anmeldenummer: PCT/EP2009/055512
(87) Internationale Veröffentlichungsnummer: WO 2009/149996

(56) Entgegenhaltungen:
- EP-A- 1 749 572
- WO-A-92/10450
- WO-A-2004/052809
- WO-A-2005/019139
- A. BAYLET, S. ROYER, P. MARÉCOT, J.M. TATIBOUËT, D. DUPREZ: "High catalytic activity and stability of Pd doped hexaaluminate catalysts for the CH4 catalytic combustion" APPLIED CATALYSIS B: ENVIRONMENTAL, Bd. 77, 1. August 2007 (2007-08-01), Seiten 237-247, XP002539272

## Beschreibung

Die vorliegende Erfindung betrifft einen Katalysator und ein Verfahren zur Herstellung von gesättigten Ethern durch Hydrierung ungesättigter Ether, insbesondere zur Herstellung von Alkoxyoctanen und Alkoxydimethyloctanen durch Hydrierung von Octadienylalkylethern oder Dimethyloctadienylalkylether.

Alkoxyverbindungen von Octanen oder Dimethyloctanen sind Vorstufen für die Herstellung von Octenen oder Dimethyloctenen. 1-Alkoxyoctan kann beispielsweise als Vorstufe für die Herstellung von 1-Octen, das als Comonomer zur Modifizierung von Polyethylen und Polypropylen verwendet wird, eingesetzt werden. Es ist bekannt, Octadienylalkylether und Dimethyloctadienylalkylether durch Umsetzung von 1,3-Butadien oder Isopren mit Alkoholen (Telomerisation) herzustellen.

Die Hydrierung der genannten ungesättigten Ether zu den entsprechenden gesättigten Ethern ist an sich bekannt. In WO 2005/019139 wird die Hydrierung von Octadienylethern zu den entsprechenden gesättigten Ethern, insbesondere die Hydrierung von 1-Methoxy-2,7-octadien, beschrieben. Die Hydrierung wird in Gegenwart eines Trägerkatalysators, bestehend aus 5 Massen-% Palladium auf Bariumsulfat, im Temperaturbereich 0 bis 100 °C und in einem Druckbereich von 1 bis 25 bar durchgeführt. In der Beschreibung wird ausgeführt, dass bei der Hydrierung Lösemittel wie beispielsweise Ether, aromatische Kohlenwasserstoffe, Paraffine, halogenierte Kohlenwasserstoffe und Nitrile eingesetzt werden können. In den Beispielen wird die Hydrierung ohne Verwendung eines Lösemittels durchgeführt.

In EP 0 561 779 wird ein Verfahren zur Hydrierung von Octadienylethern beschrieben, bei dem als Hydrierkontakte u. a. Trägerkatalysatoren, bestehend aus 0,1 bis 10 Massen-% Palladium auf γ-Aluminiumoxid, verwendet werden. Die Hydrierung wird im Temperaturbereich von 50 bis 200°C und im Druckbereich von 0,1 bis 100 bar durchgeführt. Optional kann die Hydrierung in Gegenwart eines Lösemittels, z. B. eines Alkohols, durchgeführt werden. Im Beispiel wird 99,3%iges 1-Methoxy-2,7-octadien bei 80 °C und 15 bar an einem Trägerkatalysator, bestehend aus 0,3 Massen-% Palladium auf γ-Aluminiumoxid, mit Wasserstoff in Abwesenheit eines Lösemittels hydriert. Die Ausbeute an gesättigtem Ether beträgt praktisch 100 %. Details zu den Katalysatoren werden nicht genannt genannt, so dass angenommen werden muss, dass alle derartigen Palladium-γ-Aluminiumoxid-Trägerkatalysatoren für die Hydrierung von Alkoxyoctadienylether zu den entsprechenden gesättigten Ethern geeignet sind.

Bei der Herstellung von Alkoxyoctadienylderivaten durch Umsetzung von Butadien oder Isopren mit einem Alkohol (Telomerisation) entstehen Produktgemische, die noch Einsatzalkohol enthalten. Die vollständige Abtrennung des überschüssigen Alkohols ist oft aufwändig. Sollen aus dem Telomerisationsprodukt durch Hydrierung der beiden olefinischen Doppelbindungen und anschließender Alkoholabspaltung ohnehin Octene oder Dimethyloctene hergestellt werden, können die Abtrennung eines Teils des im Telomerisationsprodukt vorhandenen Alkohols und die Abtrennung des bei der Spaltung des Hydrierproduktes entstandenen Alkohols in einem gemeinsamen Aufarbeitungsschritt erfolgen. Dieses Vorgehen ist allerdings nur dann vorteilhaft, wenn während der Hydrierung aus dem Alkohol keine Nebenprodukte entstehen. Ansonsten würde die Bildung von Nebenprodukten, beispielsweise die Bildung von Dimethylether aus Methanol, nicht nur den Aufarbeitungsaufwand, sondern auch die spezifischen Stoffkosten erhöhen.

Die bekannten und bislang in derartigen Verfahren eingesetzten Trägerkatalysatoren haben weiterhin den Nachteil, dass deren Aktivität relativ rasch nachlässt und diese somit keine ausreichend anhaltende Langzeit-Aktivität aufweisen.

Die Aufgabe der vorliegenden Erfindung bestand deshalb in der Bereitstellung eines Katalysators, mit dem Octadienylether-Gemische in Gegenwart von Alkohol ohne Bildung von Nebenprodukten mit Wasserstoff zu den entsprechenden Octylethern hydriert werden können, und der insbesondere eine hohe Langzeit-Aktivität aufweist. Es wurde nun gefunden, dass Trägerkatalysatoren auf Basis von Palladium-γ-Aluminiumoxid, besonders gut für die selektive Hydrierung von mehrfach ungesättigten Ethern, wie insbesondere Octadienylethern und deren Gemische zu den entsprechenden gesättigten Ethern, wie insbesondere Octylethern geeignet sind, wenn das Katalysatorträgermaterial 1 bis 1000 Massen-ppm Natriumoxid enthält und ein spezifisches Porenvolumen von 0,4 bis 0,9 ml/g und eine BET-Oberfläche von 150 bis 350 m²/g aufweist.

Gegenstand der vorliegenden Erfindung ist daher ein Trägerkatalysator auf Basis von Palladium-γ-Aluminiumoxid, welcher dadurch gekennzeichnet ist, dass das Katalysatorträgermaterial 1 bis 1000 Massen-ppm Natriumoxid enthält und ein spezifisches Porenvolumen von 0,4 bis 0,9 ml/g und eine BET-Oberfläche von 150 bis 350 m²/g aufweist, sowie ein Verfahren zu dessen Herstellung.

Gegenstand der vorliegenden Erfindung ist weiterhin ein Verfahren zur Herstellung von gesättigten Ethern durch Hydrierung ungesättigter Ether, in dem als Katalysator ein Trägerkatalysator auf Basis von Palladium-γ-Aluminiumoxid eingesetzt wird, welcher dadurch gekennzeichnet ist, dass das Katalysatorträgermaterial 1 bis 1000 Massen-ppm Natriumoxid enthält und ein spezifisches Porenvolumen von 0,4 bis 0,9 ml/g und eine BET-Oberfläche von 150 bis 350 m²/g aufweist.

Die vorliegende Erfindung zeigt die folgenden unerwarteten Vorteile: Bei der Hydrierung von Octadienylethern zu den entsprechenden gesättigten Ethern stören Begleitstoffe nicht. So wird durch den erfindungsgemäßen Katalysator die Bildung von Ethern durch Wasserabspaltung aus eventuell vorhandenen Alkoholen bei der Hydrierung nicht gefördert. Im Gemisch in geringen Konzentrationen eventuell vorhandene Hochsieder bewirken keine signifikante Verschlechterung der Hydrieraktivität des Katalysators. Ein besonderer Vorteil der Erfindung liegt darin, dass der Katalysator eine lange Standzeit aufweist und die Hydrierselektivität während der Laufzeit praktisch konstant bleibt. Dies ist vor allem deshalb überraschend, weil, wie Beispiel 2 zeigt, übliche Palladium-γ-Aluminiumoxid-Katalysatoren diese Leistung nicht erbringen.

Das erfindungsgemäße Verfahren sowie die erfindungsgemäßen Katalysatoren werden nachfolgend näher beschrieben.

Der erfindungsgemäße Trägerkatalysator auf Basis von Palladium-γ-Aluminiumoxid ist dadurch charakterisiert, dass das zugrunde liegende γ-Aluminiumoxid-Trägermaterial 1 bis 1000 Massen-ppm Natriumoxid enthält und ein spezifisches Porenvolumen von 0,4 bis 0,9 ml/g und eine BET-Oberfläche von 150 bis 350 m²/g aufweist.

Zur Herstellung des erfindungsgemäßen Trägerkatalysators wird ein Trägermaterial auf Basis von γ-Aluminiumoxid verwendet, welches 1 bis 1000 Massen-ppm Natriumverbindungen (berechnet als Natriumoxid) enthält. Vorzugsweise enthält das Trägermaterial 1 bis 750 Massen-ppm, insbesondere 1 bis 500 Massen-ppm Natriumverbindungen (jeweils berechnet als Natriumoxid).

Optional kann das Trägermaterial Sulfat bzw. Sulfatgruppen und/oder Siliziumdioxid enthalten. Der Sulfatanteil kann bis zu 1500 Massen-ppm betragen. Weiterhin kann das Trägermaterial bis zu 20 Massen-% Siliziumdioxid enthalten.

Die BET-Oberfläche des verwendeten Trägermaterials beträgt 150 bis 350 m²/g, bevorzugt 200 bis 320 m²/g, besonders bevorzugt 220 bis 300 m²/g (bestimmt nach dem BET-Verfahren durch Stickstoff-Adsorption gemäß DIN 9277).

Das Porenvolumen des Trägermaterials beträgt 0,4 bis 0,9 ml/g (bestimmt durch Quecksilber-Intrusion nach DIN 66133).

Der mittlere Porenradius des Trägermaterials beträgt vorzugsweise 2 bis 50 nm, besonders bevorzugt 5 bis 30 nm und insbesondere 7 bis 15 nm (bestimmt durch Kombination der Porengrößenbestimmung nach DIN 66133 und Bestimmung der Mesoporen nach BJH gemäß DIN 66134).

Geeignete derartige γ-Aluminiumoxid-Trägermaterialien sind beliebig kommerziell erhältlich.

Der erfindungsgemäße Trägerkatalysator enthält als hydrieraktive Komponente Palladium. Der Palladiumgehalt im einsatzbereiten Katalysator beträgt vorzugsweise 0,1 bis 10 Massen-%, insbesondere 0,1 bis 3 Massen-% und besonders bevorzugt 0,2 bis 1 Massen-%.

Der erfindungsgemäße Katalysator kann in der Weise hergestellt werden, in dem eine oder mehrere Palladiumverbindung(en) auf ein wie vorstehend beschriebenes Trägermaterial aufgebracht wird. Das Aufbringen kann durch Tränken des Trägers mit einer Palladiumverbindung enthaltenden Lösung, Aufsprühung von Palladiumverbindungen enthaltenden Lösungen auf den Träger oder durch andere gleichwirkende Verfahren erfolgen. Als Palladiumverbindungen, die auf den Träger aufgebracht werden können, eignen sich beispielsweise Palladiumacetat, Palladiumacetylacetonat, Palladiumchlorid, Palladiumnitrat-Dihydrat oder Palladiumsulfat-Dihydrat, wobei Palladiumnitrat-Dihydrat die bevorzugte Verbindung ist. Als Palladiumverbindungen aufweisende Lösungen werden bevorzugt wässrige Palladiumsalzlösung eingesetzt. Vorzugsweise weisen solche Lösungen einen Gehalt an Palladium von 1 bis 15 Massen-%, bevorzugt von 5 bis 10 Massen-% auf.

Nach dem Aufbringen der Palladiumverbindung(en) wird das Trägermaterial, typischerweise bei Temperaturen von 80 bis 150 °C, getrocknet und optional bei Temperaturen von 200 bis 600 °C kalziniert.

In einer besonderen Ausführungsform kann die Aufbringung der, Palladiumverbindung(en) Trocknung und gegebenenfalls Kalzinierung in einem Arbeitsgang erfolgen. So kann etwa der erfindungsgemäße Trägerkatalysator durch Aufsprühen einer Lösung einer Palladiumverbindung auf das Trägermaterial bei einer Temperatur von 80 °C oder höher erhalten werden.

Die erfindungsgemäßen Trägerkatalysatoren werden bevorzugt durch Aufsprühen einer wässrigen Lösung mit Palladiumsalzverbindungen auf das Trägermaterial bei Temperaturen von 10 bis 170 °C, insbesondere von 50 bis 150 °C und optional anschließender Kalzinierung im Temperaturbereich von 170 bis 550 °C, insbesondere von 200 bis 450 °C hergestellt. Wird das Aufsprühen bei Normaldruck vorgenommen, beträgt die Temperatur des zu besprühenden Materials vorzugsweise 100 bis 170 °C. Wird das Aufsprühen im Vakuum durchgeführt, wobei der Druck vorzugsweise kleiner als der Wasserdampfpartialdruck der Sprühlösung ist, beträgt die Temperatur vorzugsweise von 20 bis 100 °C.

Bei der Aufsprühung verdampft der größte Teil des in der Sprühlösung vorhandenen Wassers Dadurch wird erreicht, dass sich das Palladium auf dem Trägermaterial in einer Randschicht, die eine Dicke von 50 bis 300 µm umfasst, befindet. Typischerweise befinden sich etwa 90 % des aufgebrachten Palladiums in dieser Randschicht.

Die erfindungsgemäßen Trägerkatalysatoren werden vorzugsweise in einer Form hergestellt, die bei der Hydrierung einen geringen Strömungswiderstand bietet. Typische Formen sind etwa Tabletten, Zylinder, Strangextrudate oder Ringe. Die Formgebung erfolgt dabei im Regelfall am Trägermaterial vor der Aufbringung der Palladiumverbindung. Es können auch granulatförmige Träger zur Herstellung der Trägerkatalysatoren eingesetzt werden. Durch Aussieben kann dabei ein Katalysatorträger mit der gewünschten Korngröße abgetrennt werden. Häufig können γ-Aluminiumoxid oder γ-Aluminiumoxid enthaltende Trägermaterialien bereits als entsprechende Formkörper bezogen werden.

Das erfindungsgemäße Verfahren zur Herstellung von gesättigten Ethern durch Hydrierung ungesättigter Ether zeichnet sich dadurch aus, dass als Katalysator ein wie vorstehend charakterisierter Trägerkatalysator auf Basis von Palladium-γ-Aluminiumoxid eingesetzt wird. In dem erfindungsgemäßen Verfahren können Mischungen eingesetzt werden, die mehrfach ungesättigte Ether sowie Alkohol, vorzugsweise Methanol, Ethanol und/oder Propanol enthalten. Das molare Verhältnis von Alkohol zu mehrfach ungesättigtem Ether im Edukt-Gemisch beträgt typischerweise 2 zu 98 bis 40 zu 60, insbesondere 5 zu 95 bis 25 zu 75 und besonders bevorzugt 10 zu 90 bis 22 zu 78.

Das Verfahren kann kontinuierlich oder diskontinuierlich durchgeführt werden. Vorzugsweise wird das Verfahren kontinuierlich durchgeführt. Die Hydrierung kann an im Festbett angeordneten erfindungsgemäßen Trägerkatalysatoren durchgeführt werden.

Im erfindungsgemäßen Verfahren kann die Hydrierung in flüssiger Phase oder in der Gasphase durchgeführt werden. Vorzugsweise wird im erfindungsgemäßen Verfahren eine kontinuierliche Hydrierung an einem im Festbett angeordneten erfindungsgemäßen Trägerkatalysator, bei dem sich unter Reaktionsbedingungen die Produkt/Eduktphase hauptsächlich im flüssigen Zustand befindet, durchgeführt.

Wenn die Hydrierung kontinuierlich an einem im Festbett angeordneten Katalysator durchgeführt wird, ist es zweckmäßig, den Trägerkatalysator vor der Hydrierung in die aktive Form zu überführen. Dies kann durch Reduktion des Trägerkatalysators mit wasserstoffhaltigen Gasen bei programmgesteuerter Temperaturführung erfolgen. Beispielsweise wird der Katalysator im H₂-Strom mit 5 K/min bis auf 200 °C aufgeheizt, für 2 h die Temperatur gehalten und dann auf Reaktionstemperatur abgesenkt. Dabei kann die Reduktion gegebenenfalls in Gegenwart einer flüssigen Phase, die über den Katalysator rieselt, durchgeführt werden. Als flüssige Phase kann dabei ein Lösemittel oder bevorzugt das Hydrierprodukt eingesetzt werden.

Für das erfindungsgemäße Verfahren können unterschiedliche Verfahrensvarianten gewählt werden. Es kann adiabatisch, polytrop oder praktisch isotherm, d. h. mit einem Temperaturanstieg von typischerweise kleiner als 10 °C, ein- oder mehrstufig durchgeführt werden. Im letzteren Falle kann man alle Reaktoren, vorzugsweise Rohrreaktoren, adiabatisch oder praktisch isotherm sowie einen oder mehrere adiabatisch und die anderen praktisch isotherm betreiben. Weiterhin ist es möglich, die gesättigten Verbindungen im geraden Durchgang oder unter Produktrückführung zu hydrieren.

Das erfindungsgemäße Verfahren wird bevorzugt in der Flüssig/Gas-Mischphase oder Flüssigphase in Dreiphasenreaktoren im Gleichstrom durchgeführt, wobei das Hydriergas in an sich bekannter Weise im flüssigen Edukt/Produktstrom verteilt wird. Im Interesse einer gleichmäßigen Flüssigkeitsverteilung, einer verbesserten Reaktionswärmeabfuhr und einer hohen Raum-Zeit-Ausbeute werden die Reaktoren meist mit hohen Flüssigkeitsbelastungen von 15 bis 120, insbesondere von 25 bis 80 m³ pro m² Querschnitt des leeren Reaktors und Stunde betrieben. Wird ein Reaktor im geraden Durchgang betrieben, so kann die spezifische Katalysatorbelastung (LHSV) Werte zwischen 0,1 und 10 h⁻¹ annehmen.

Die Hydrierung kann in Ab- oder in Anwesenheit eines Lösemittels durchgeführt werden. Vorzugsweise wird die Hydrierung in Gegenwart eines Lösemittels durchgeführt. Durch die Verwendung eines Lösemittels kann die Konzentration des zu hydrierenden mehrfach ungesättigten Ethers im Reaktorzulauf begrenzt werden, wodurch eine bessere Temperaturkontrolle im Reaktor erreicht wird. Auf diese Weise wird eine Minimierung von Nebenreaktionen und somit eine Erhöhung der Produktausbeute erreicht. Bevorzugt wird die Konzentration des zu hydrierenden mehrfach ungesättigten Ethers im Reaktorzulauf, bei der Verwendung mehrerer Reaktoren insbesondere im Zulauf zum ersten Reaktor, auf eine Konzentration von 1 bis 35 Massen-%, besonders bevorzugt 5 bis 25 Massen-% eingestellt. Die gewünschte Konzentration des zu hydrierenden mehrfach ungesättigten Ethers im Reaktorzulauf kann bei Reaktoren, die in Schlaufenfahrweise betrieben werden, durch das Kreislaufverhältnis (Mengenverhältnis vom zurückgeführten Hydrieraustrag zu Edukt) eingestellt werden.

Als Lösemittel können alle Flüssigkeiten eingesetzt werden, die mit dem Edukt und Produkt eine homogene Lösung bilden, sich unter Hydrierbedingungen inert verhalten und sich leicht vom Produkt abtrennen lassen. Das Lösemittel kann auch ein Gemisch mehrerer Stoffe sein und gegebenenfalls Wasser enthalten. Bevorzugt wird als Lösemittel ein gesättigter Ether eingesetzt, wie er etwa im erfindungsgemäßen Verfahren als Hydrierprodukt erhalten wird. Auf diese Weise kann ein aufwändiger Schritt, bei dem das Lösemittel wieder aus dem Produktaustrag entfernt wird, vermieden werden.

Das erfindungsgemäße Verfahren wird vorzugsweise bei einem Druck von 20 bis 150 bar, bevorzugt bei 30 bis 120 bar und besonders bevorzugt bei 40 bis 100 bar durchgeführt. Die Hydriertemperatur, bei der das Verfahren durchgeführt wird, beträgt vorzugsweise 50 und 150 °C, insbesondere 60 bis 120 °C.

Als Hydriergase können Wasserstoff oder beliebige wasserstoffhaltige Gase bzw. Gasgemische eingesetzt werden. Die eingesetzten Gase sollten keine schädlichen Mengen an Katalysatorgiften wie beispielsweise Kohlenmonoxid oder Schwefelwasserstoff enthalten. Bevorzugt werden Gase eingesetzt, die weder Kohlenmonoxid noch Schwefelwasserstoff enthalten. Neben Wasserstoff können die eingesetzten Gase ein oder mehrere Inertgas(e). Enthalten. Inertgasbestandteile können z. B. Stickstoff oder Methan sein. Bevorzugt wird als wasserstoffhaltiges Gas Wasserstoff in einer Reinheit von größer 95 Volumen-%, insbesondere von größer 98 Volumen-% eingesetzt.

Wasserstoff wird im stöchiometrischen Überschuss eingesetzt. Vorzugsweise beträgt der Überschuss mehr als 10 %.

Mittels des erfindungsgemäßen Verfahrens können mehrfach ungesättigte Ether zu den entsprechenden gesättigten Ethern hydriert werden. Vorzugsweise werden mit dem erfindungsgemäßen Verfahren Alkyl-substituierte oder unsubstituierte Octadienylalkylether zu den entsprechenden Alkyl-substituierten oder unsubstituierten gesättigten Octylalkylethern hydriert. Die Alkyl-Gruppe kann z. B. eine Methyl-, Ethyl- oder Propyl-Gruppe sein. Besonders bevorzugt ist die Alkyl-Gruppe eine Methyl-Gruppe. Ganz besonders bevorzugt wird mit dem erfindungsgemäßen Verfahren 1-Methoxy-2,7-octadien zu 1-Methoxyoctan hydriert.

Die genannten Einsatzstoffe können beispielsweise durch Telomerisation erhalten werden. Bei der Telomerisation werden zwei Mol Dien mit einem Mol Alkohol umgesetzt. Bei der Telomerisation von Isopren entstehen

Dimethyloctadienylalkylether, bei der Telomerisation von Butadien Octadienylalkylether und bei der gekreuzten Telomerisation von Isopren und Butadien ein Gemisch aus Dimethyloctadienylalkylethern, Methyloctadienylalkylether und Octadienylalkylethern. Als Alkohole können bei der Telomerisation insbesondere Methanol, Ethanol oder Propanol eingesetzt werden. Bevorzugt wird bei der Telomerisation Methanol als Alkohol eingesetzt.

Bevorzugte Einsatzstoffe sind Alkyl-substituierte oder unsubstituierte Octadiene mit einer endständigen Alkoxygruppe, insbesondere einer Methoxygruppe. Ein ganz besonders bevorzugter Einsatzstoff ist 1-Methoxyocta-2,7-dien. Verfahren zur Herstellung dieser Verbindung sind beispielsweise in DE 101 49 348, DE 103 12 829, DE 10 2005 036039.4, DE 10 2005 036038.6, DE 10 2005 036040.8 beschrieben.

Die für die erfindungsgemäße Hydrierung verwendeten Einsatzstoffe müssen keine Reinstoffe sein, sondern können noch weitere Komponenten enthalten. Beispielsweise enthält ein durch Telomerisation hergestelltes 1-Methoxy-octa-2,7-dien (1-MODE) häufig einige Massen-% an 3-Methoxy-octa-2,7-dien. Weiterhin können auch andere Doppelbindungsisomere vorhanden sein. Technische Gemische können darüber hinaus Methanol, Lösemittel und Nebenprodukte aus der Telomerisation enthalten.

Die bei der erfindungsgemäßen Hydrierung erhaltenen Reaktionsgemische können als solche verwendet werden oder aufgearbeitet werden, beispielsweise durch Destillation.

Aus dem durch Hydrierung hergestellten gesättigten Ether können durch Alkoholabspaltung Monoolefine gewonnen werden. So kann z. B. 1-Methoxyoctan (1-MOAN) zu 1-Octen umgesetzt werden. Ein solches Verfahren wird etwa in DE 102 57 499 beschrieben.

Die folgenden Beispiele sollen die Erfindung erläutern, ohne sie darauf zu beschränken.

### Beispiel 1: Herstellung eines Palladium-γ-Aluminiumoxid-Katalysators (nicht erfindungsgemäß)

Ein Aluminiumoxidträger (CPN der Fa. Alcoa) wurde mit einer Pd-Nitrat-haltigen wässrigen Lösung (Pd-Gehalt 15 Massen-%) besprüht und anschließend bei 120 °C 2 h getrocknet. Anschließend erfolgte eine Reduktion im wasserstoffhaltigen Stickstoffstrom bei 200 °C für 2 h.

Der Aluminiumoxidträger bestand aus einem Granulat mit einer mittleren Partikelgröße von 1,2 bis 2,4 mm (bestimmt durch Siebanalyse) und hatte eine BET-Oberfläche von ca. 250 m²/g, ein Porenvolumen von 0,33 ml/g und einen Natriumoxidgehalt von 0,5 Massen-% (jeweils Lieferantenangaben). Die Eindringtiefe des abgeschiedenen Pd betrug (gemäß EDX-Analyse) ca. 100 bis 250 µm. Der Palladiumgehalt bezogen auf die gesamte Katalysatormasse betrug ca. 0,5 Massen-%.

### Beispiel 2: Hydrierung von 1-Methoxy-2,7-octadien (MODE) (nichterfindungsgemäß)

I.) In einem Rührkesselautoklav mit einem Reaktionsvolumen von 1,4 l wurden in einem Korb 60 g des Katalysators eingebracht. Der Autoklav wurde mit 1,4 l einer Mischung aus 80 Massen-% MODE und 20 Massen-% Methanol befüllt. Nach einer Inertisierung mit Stickstoff wurde der Reaktor auf 80 °C beheizt und anschließend mit Wasserstoff auf einen Druck von 15 bar absolut gebracht. Zum Start der Reaktion wurde der Begasungsrührer auf eine Umdrehungszahl von 1000 min⁻¹ eingestellt. Zur Beobachtung des Reaktionsverlaufs wurden in regelmäßigen Abständen Proben gezogen und per Gaschromatograph analysiert.

Nach einer Reaktionszeit von 2 h war der Umsatz vollständig. Der Gehalt des Produkts 1-MOAN betrug 77,5 GC-Flächen-%. Anschließend wurde der Autoklav entleert, der Katalysator wurde im Reaktor belassen. Die Laufzeit des Katalysators betrug insgesamt 7 h.

II.) Der Katalysator mit einer Laufzeit von 7 h wurde nach Versuchsbeispiel 2.l im Autoklav belassen. Der Autoklav wurde mit 1,4 l einer Mischung aus 98 Massen-% MODE und 2 Massen-% Methanol befüllt. Nach einer Inertisierung mit Stickstoff wurde der Reaktor auf 80 °C beheizt und anschließend mit Wasserstoff auf einen Druck von 15 bar absolut gebracht. Zum Start der Reaktion wurde der Begasungsrührer auf eine Umdrehungszahl von 1000 min⁻¹ eingestellt. Zur Beobachtung des Reaktionsverlaufs wurden in regelmäßigen Abständen Proben gezogen und per Gaschromatograph analysiert.

Nach einer Reaktionszeit von 4 h lag die Konzentration von 1-MOAN bei 94,5 GC-Flächen-%. Anschließend wurde der Autoklav entleert, der Katalysator wurde im Reaktor belassen.

III.) Wiederholung von Versuch I mit dem in Versuch I und II gebrauchten Katalysator nach einer gesamten Laufzeit von 11 h. Der Autoklav wurde mit 1,4 l einer Mischung aus 80 Massen-% MODE und 20 Massen-% Methanol befüllt. Nach einer Inertisierung mit Stickstoff wurde der Reaktor auf 80 °C beheizt und anschließend mit Wasserstoff auf einen Druck von 15 bar absolut gebracht. Zum Start der Reaktion wurde der Begasungsrührer auf eine Umdrehungszahl von 1000 min⁻¹ eingestellt. Zur Beobachtung des Reaktionsverlaufs wurden in regelmäßigen Abständen Proben gezogen und per Gaschromatograph analysiert.

Nach einer Reaktionszeit von 4 h war der Umsatz unvollständig. Der Gehalt des Produkts 1-MOAN betrug 42,5 GC-Flächen-%. Der Vergleich der Versuche I und III zeigte einen starken Rückgang der MOAN-Bildung durch Katalysatordesaktivierung.

### Beispiel 3: Herstellung eines Palladium-γ-Aluminiumoxid-Katalysators (erfindungsgemäß)

Ein Aluminiumoxidträger (SP 538 E, Fa. Axens) wurde mit einer Pd-Nitrat-haltigen wässrigen Lösung (Pd-Gehalt 15 Massen-%) bei 100 °C besprüht und anschließend bei 450 °C für 60 min. getempert. Zur Aktivierung erfolgte eine Reduktion im Wasserstoffstrom bei 250 °C über 2 h.

Der Aluminiumoxidträger bestand aus einem Extrudat in der Form von Zylindern mit einem Durchmesser von 1,2 mm und Längen, die zwischen 2 und 6 mm betrugen, und hatte eine BET-Oberfläche von ca. 280 m²/g (bestimmt nach dem BET-Verfahren durch Stickstoff-Adsorption gemäß DIN 9277), ein Porenvolumen von 0,72 ml/g (Lieferantenangabe), einen Natriumoxidgehalt von 0,03 Massen-% (Lieferantenangabe) und einen Sulfatgehalt von ca. 0,1 Massen-% (Lieferantenangabe) . Die Eindringtiefe des abgeschiedenen Pd betrug ca. 80 bis 150 µm und der Palladiumgehalt betrug bezogen auf die gesamte Katalysatormasse ca. 0,5 Massen-% (jeweils bestimmt mittels EDX-Analyse bei einer Untersuchung des Katalysatorkornquerschnitts mit dem Raster-Elekronen-Mikroskop).

### Beispiel 4: Hydrierung von 1-Methoxy-2,7-octadien (MODE) (erfindungsgemäß)

l.) In einem Rührkesselautoklav mit einem Reaktionsvolumen von 1,4 l wurden in einem Korb 60 g des erfindungsgemäßen Katalysators (aus Beispiel 3) eingebracht. Der Autoklav wurde mit 1,4 l einer Mischung aus 80 Massen-% MODE und 20 Massen-% Methanol befüllt. Nach einer Inertisierung mit Stickstoff wurde der Reaktor auf 80 °C beheizt und anschließend mit Wasserstoff auf einen Druck von 15 bar absolut gebracht. Zum Start der Reaktion wurde der Begasungsrührer auf eine Umdrehungszahl von 1000 min⁻¹ eingestellt. Zur Beobachtung des Reaktionsverlaufs wurden in regelmäßigen Abständen Proben gezogen und per Gaschromatograph analysiert.

Nach einer Reaktionszeit von 2 h war der Umsatz unvollständig. Der Gehalt des Produkts 1-MOAN betrug 76,3 GC-Flächen-%. Anschließend wurde der Autoklav entleert, der Katalysator wurde im Reaktor belassen. Die gesamte Laufzeit des Katalysators betrug 7 h.

II.) Der Katalysator mit einer Laufzeit von 7 h wurde nach Versuchsbeispiel 2.l im Autoklav belassen. Der Autoklav wurde mit 1,4 l einer Mischung aus 98 Massen-% MODE und 2 Massen-% Methanol befüllt. Nach einer Inertisierung mit Stickstoff wurde der Reaktor auf 80°C beheizt und anschließend mit Wasserstoff auf einen Druck von 15 bar absolut gebracht. Zum Start der Reaktion wurde der Begasungsrührer auf eine Umdrehungszahl von 1000 min⁻¹ eingestellt. Zur Beobachtung des Reaktionsverlaufs wurden in regelmäßigen Abständen Proben gezogen und per Gaschromatograph analysiert.

Nach einer Reaktionszeit von 2 h lag die Konzentration von 1-MOAN bei 93,7 GC-Flächen-% und blieb auch nach 4 h unverändert. Anschließend wurde der Autoklav entleert, der Katalysator wurde im Reaktor belassen. Die Laufzeit in diesem Versuch betrug 4 h.

III.) Der Versuch gemäß I.) wurde unter Verwendung des in I. und II. benutzten Katalysators wiederholt. Die gesamte Laufzeit des Katalysators betrug zu Versuchsbeginn 11 h. In einem Rührkesselautoklav mit einem Reaktionsvolumen von 1,4 l wurden in einem Korb 60 g des in I. und II. verwendeten Katalysators eingebracht. Der Autoklav wurde mit 1,4 l einer Mischung aus 80 Massen-% MODE und 20 Massen-% Methanol befüllt. Nach einer Inertisierung mit Stickstoff wurde der Reaktor auf 80 °C beheizt und anschließend mit Wasserstoff auf einen Druck von 15 bar absolut gebracht. Zum Start der Reaktion wurde der Begasungsrührer auf eine Umdrehungszahl von 1000 min⁻¹ eingestellt. Zur Beobachtung des Reaktionsverlaufs wurden in regelmäßigen Abständen Proben gezogen und per Gaschromatograph analysiert.

Nach einer Reaktionszeit von 2 h war der Umsatz unvollständig. Der Gehalt des Produkts 1-MOAN betrug 72,5 GC-Flächen-%.

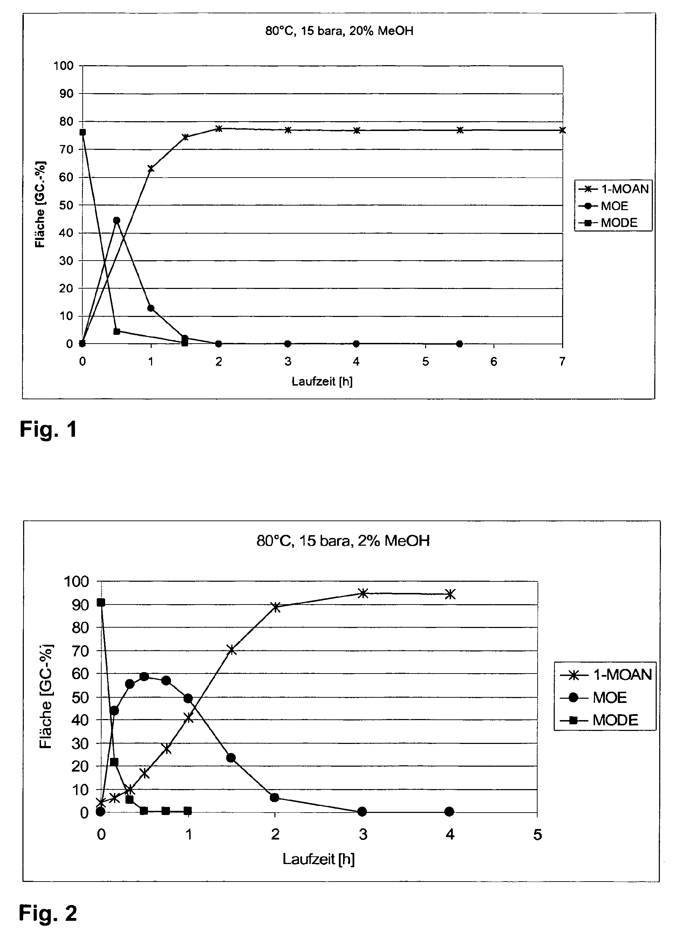

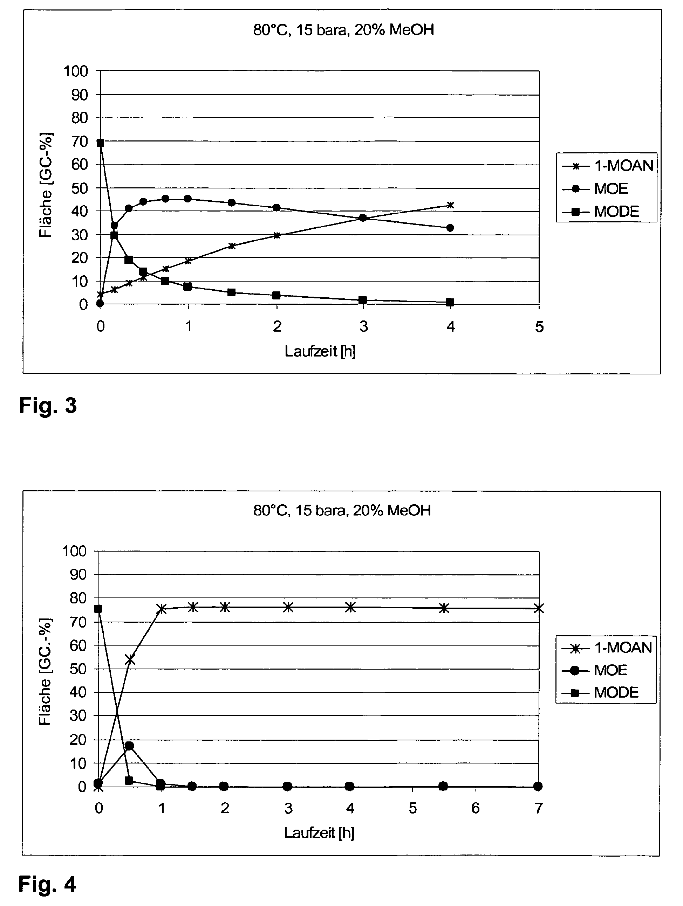

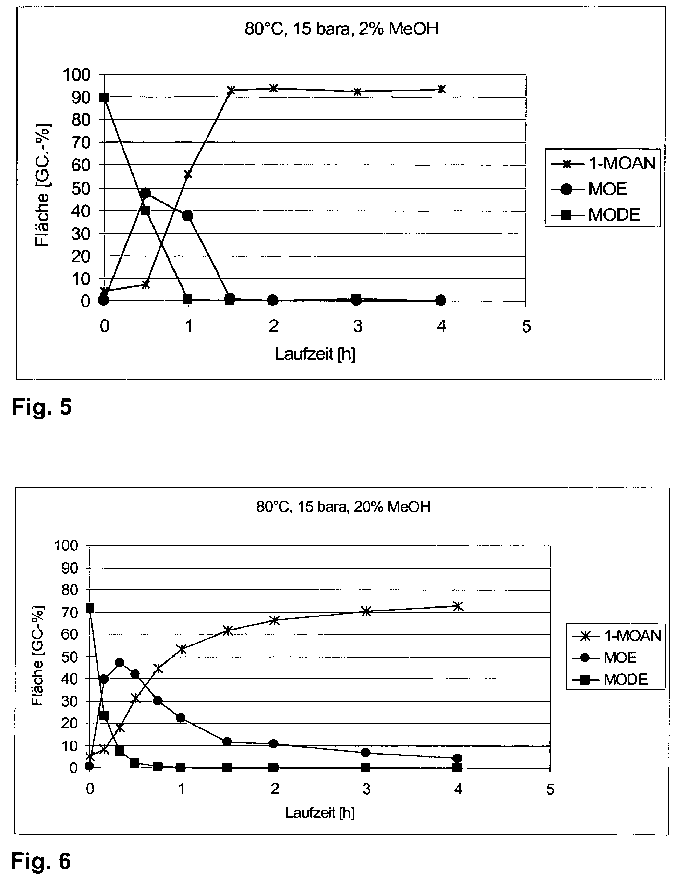

Der erfindungsgemäße Katalysator zeigte bei niedrigen MeOH-Konzentrationen eine erheblich höhere Hydrierleistung als der nicht erfindungsgemäße Katalysator (Vergleich: Fig. 2 und Fig. 5). Außerdem zeigte der erfindungsgemäße Katalysator im Wiederholungsversuch eine deutlich geringere Alterung als der nichterfindungsgemäße Katalysator (Vergleich Fig. 3 und Fig. 6).

### Beispiel 5: Langzeitversuch 1 (erfindungsgemäß):

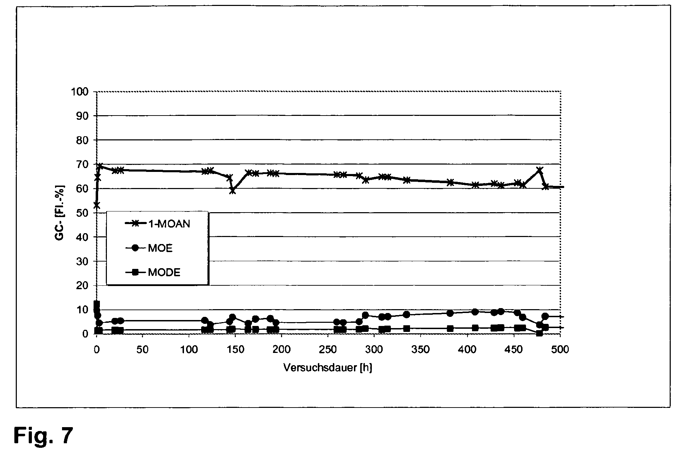

25 g des erfindungsgemäßen Katalysators gemäß Beispiel 3 und wurden in einem Rohrreaktor, der Teil einer Hydrier-Kreislaufapparatur war, platziert. Der Reaktor wurde auf 90 °C temperiert und der Katalysator für 2 h mit Wasserstoff reduziert. Anschließend wurden in die Apparatur 989 g (1200ml) einer methanolischen MODE-Lösung gefüllt. Die Methanolkonzentration betrug 20 Massen-%. Anschließend wurde bei 90 °C und 15 bara hydriert. Es wurden 70 g/h der Einsatzlösung zudosiert und unter Konstanthaltung des Reaktorvolumens eine entsprechende Menge ausgetragen. Nach 20 h wurde der quasistationäre Zustand erreicht. Die MOAN-Konzentration am Reaktorausgang betrug 67 %. Nach 500 h betrug die MOAN-Konzentration immer noch 61 %. Die Veränderungen der GC-Flächenanteile über die Versuchsdauer sind in Fig. 7 dargestellt.

In den Figuren Fig. 1 bis Fig. 7 sind Diagramme dargestellt, in denen der Verlauf der GC-Flächen über die Reaktionszeiten dargestellt ist.

In den Diagrammen bedeuten:
- MODE:: 1-Methoxyocta-2,7-dien
- MOE:: 1-Methoxyocten (Summe der einzelnen Isomere, die sich durch Lage und/oder Konfiguration der Doppelbindung unterscheiden)
- MOAN:: 1-Methoxyoctan

In Fig. 1 ist der Verlauf der GC-Flächenverhältnisse beim Versuch gemäß Beispiel 2, I.) dargestellt.

In Fig. 2 ist der Verlauf der GC-Flächenverhältnisse beim Versuch gemäß Beispiel 2, II.) dargestellt.

In Fig. 3 ist der Verlauf der GC-Flächenverhältnisse beim Versuch gemäß Beispiel 2, III.) dargestellt.

In Fig. 4 ist der Verlauf der GC-Flächenverhältnisse beim Versuch gemäß Beispiel 4,

I.) dargestellt.

In Fig. 5 ist der Verlauf der GC-Flächenverhältnisse beim Versuch gemäß Beispiel 4, II.) dargestellt.

In Fig. 6 ist der Verlauf der GC-Flächenverhältnisse beim Versuch gemäß Beispiel 4, III.) dargestellt.

In Fig. 7 ist der Verlauf der GC-Flächenverhältnisse beim Versuch gemäß Beispiel 5 dargestellt.

## Patentansprüche

1. Verfahren zur Hydrierung von mehrfach ungesättigten Ethern mit Wasserstoff zu den entsprechenden gesättigten Ethern,
wobei als mehrfach ungesättigte Ether unsubstituierte und/oder Alkyl-substituierte Octadienylalkylether eingesetzt werden und zu den entsprechenden gesättigten Ethern hydriert werden,
und wobei 1-Methoxy-2,7-octadien zu 1-Methoxyoctan hydriert wird,
**dadurch gekennzeichnet,**
**dass** als Katalysator ein Trägerkatalysator auf Basis von Palladium-γ-Aluminiumoxid eingesetzt wird,
wobei das Katalysatorträgermaterial 1 bis 1000 Massen-ppm Natriumoxid enthält und ein spezifisches Porenvolumen von 0,4 bis 0,9 ml/g und eine BET-Oberfläche von 150 bis 350 m²/g aufweist.

2. Verfahren nach Anspruch 1,
**dadurch gekennzeichnet,**
**dass** die Hydrierung in Gegenwart eines Lösemittels durchgeführt wird.

3. Verfahren nach Anspruch 2,
**dadurch gekennzeichnet,**
**dass** als Lösemittel ein gesättigter Ether eingesetzt wird, wie er bei der Hydrierung erhalten werden kann.

4. Verfahren nach zumindest einem der Ansprüche 1 bis 3,
**dadurch gekennzeichnet,**
**dass** die Konzentration des zu hydrierenden mehrfach ungesättigten Ethers im Reaktorzulauf auf eine Konzentration von 1 bis 35 Massen-% eingestellt wird.

5. Verfahren nach einem der Ansprüche 1 bis 4,
**dadurch gekennzeichnet,**
**dass** das Katalysatorträgermaterial eine BET-Oberfläche von 200 bis 320 m²/g, vorzugsweise von 220 bis 300 m²/g, aufweist.

6. Verfahren nach einem der Ansprüche 1 bis 5,
**dadurch gekennzeichnet,**
**dass** der Palladiumgehalt 0,1 bis 10 Massen-% beträgt.

7. Verfahren nach einem der Ansprüche 1 bis 6,
**dadurch gekennzeichnet,**
**dass** sich das Palladium auf dem Trägermaterial in einer Randschicht, die eine Dicke von 50 bis 300 µm umfasst, befindet.

8. Verfahren nach einem der Ansprüche 1 bis 7,
**dadurch gekennzeichnet,**
**dass** der mittlere Porenradius des Trägermaterials 2 bis 50 nm, beträgt.

9. Verfahren nach einem der Ansprüche 1 bis 8,
**dadurch gekennzeichnet,**
**dass** das Trägermaterial Sulfat und/oder Siliziumdioxid enthält.

## Claims

1. Process for hydrogenating polyunsaturated ethers with hydrogen to give the corresponding saturated ethers,
the polyunsaturated ethers used being unsubstituted and/or alkyl-substituted octadienyl alkyl ethers and being hydrogenated to the corresponding saturated ethers,
and 1-methoxy-2,7-octadiene being hydrogenated to 1-methoxyoctane,
**characterized in that**
the catalyst used is a supported catalyst based on palladium-γ-alumina,
the catalyst support material containing 1 to 1000 ppm by mass of sodium oxide and having a specific pore volume of 0.4 to 0.9 ml/g and a BET surface area of 150 to 350 m²/g.

2. Process according to Claim 1,
**characterized in that**
the hydrogenation is performed in the presence of a solvent.

3. Process according to Claim 2,
**characterized in that**
the solvent used is a saturated ether as can be obtained in the hydrogenation.

4. Process according to at least one of Claims 1 to 3,
**characterized in that**
the concentration of the polyunsaturated ether to be hydrogenated in the reactor feed is adjusted to a concentration of 1 to 35% by mass.

5. Process according to one of Claims 1 to 4,
**characterized in that**
the catalyst support material has a BET surface area of 200 to 320 m²/g, preferably of 220 to 300 m²/g.

6. Process according to one of Claims 1 to 5, **characterized in that**
the palladium content is 0.1 to 10% by mass.

7. Process according to one of Claims 1 to 6,
**characterized in that**
the palladium is present on the support material in a boundary layer which encompasses a thickness of 50 to 300 µm.

8. Process according to one of Claims 1 to 7,
**characterized in that**
the mean pore radius of the support material is 2 to 50 nm.

9. Process according to one of Claims 1 to 8,
**characterized in that**
the support material comprises sulphate and/or silicon dioxide.

## Revendications

1. Procédé d'hydrogénation d'éthers polyinsaturés avec de l'hydrogène pour former les éthers saturés correspondants,
des éthers d'octadiénylalkyle non substitués et/ou à substitution alkyle étant utilisés en tant qu'éthers polyinsaturés et hydrogénés pour former les éthers saturés correspondants,
et le 1-méthoxy-2,7-octadiène étant hydrogéné en 1-méthoxyoctane,
**caractérisé en ce qu'**un catalyseur supporté à base d'oxyde de palladium et d'aluminium γ est utilisé en tant que catalyseur,
le matériau support du catalyseur contenant 1 à 1 000 ppm en masse d'oxyde de sodium et présentant un volume poreux spécifique de 0,4 à 0,9 ml/g et une surface BET de 150 à 350 m²/g.

2. Procédé selon la revendication 1, **caractérisé en ce que** l'hydrogénation est réalisée en présence d'un solvant.

3. Procédé selon la revendication 2, **caractérisé en ce qu'**un éther saturé tel que celui pouvant être obtenu lors de l'hydrogénation est utilisé en tant que solvant.

4. Procédé selon au moins l'une quelconque des revendications 1 à 3, **caractérisé en ce que** la concentration de l'éther polyinsaturé à hydrogéner dans l'alimentation du réacteur est ajustée à une concentration de 1 à 35 % en masse.

5. Procédé selon l'une quelconque des revendications 1 à 4, **caractérisé en ce que** le matériau support du catalyseur présente une surface BET de 200 à 320 m²/g, de préférence de 220 à 300 m²/g.

6. Procédé selon l'une quelconque des revendications 1 à 5, **caractérisé en ce que** la teneur en palladium est de 0,1 à 10 % en masse.

7. Procédé selon l'une quelconque des revendications 1 à 6, **caractérisé en ce que** le palladium se trouve sur le matériau support en une couche de surface qui présente une épaisseur de 50 à 300 µm.

8. Procédé selon l'une quelconque des revendications 1 à 7, **caractérisé en ce que** le rayon de pore moyen du matériau support est de 2 à 50 nm.

9. Procédé selon l'une quelconque des revendications 1 à 8, **caractérisé en ce que** le matériau support contient du sulfate et/ou du dioxyde de silicium.
